# EUROPEAN PATENT SPECIFICATION

(11) **EP 4 463 107 B1**
(45) Date of publication and mention of the grant of the patent: **22.07.2026**
(21) Application number: 23706233.6
(22) Date of filing: 20.01.2023
(51) Int. Cl.: A61F 2/90, A61F 2/848

(54) **STENT WITH MULTIPLE KNITTING PATTERNS**
STENT MIT MEHREREN STRICKMUSTERN
STENT À MULTIPLES MOTIFS DE TRICOTAGE

(30) Priority: 21.01.2022 US 202263301731 P
(43) Date of publication of application: 20.11.2024
(73) Proprietor: Boston Scientific Scimed, Inc., Maple Grove, MN 55311 (US)
(72) Inventor: FOLAN, Martyn G., Galway (IE); GILMARTIN, Gary, Toomore Foxford F26 CK30 (IE); KEATING, Thomas Martin, Tuam, Co Galway (IE)
(74) Representative: Vossius & Partner Patentanwälte Rechtsanwälte mbB
(86) International application number: PCT/US2023/060955
(87) International publication number: WO 2023/141545

(56) References cited:
- US-A1- 2009 187 240
- US-A1- 2019 307 586
- US-A1- 2020 214 858

## Description

### CROSS-REFERENCE TO RELATED APPLICATIONS

This application claims the benefit of U.S. Provisional Patent Application Serial No. 63/301,731, filed January 21, 2022.

### TECHNICAL FIELD

The present disclosure pertains to medical devices, and methods for manufacturing and using medical devices. More particularly, the disclosure is directed to stents for implantation in a body lumen, and associated methods.

### BACKGROUND

Implantable medical devices (e.g., expandable stents) may be designed to treat a variety of medical conditions in the body. For example, some expandable stents may be designed to radially expand and support a body lumen and/or provide a fluid pathway for digested material, blood, or other fluid to flow therethrough following a medical procedure. Some medical devices may include radially or self-expanding stents which may be implanted transluminally via a variety of medical device delivery systems. These stents may be implanted in a variety of body lumens such as coronary or peripheral arteries, the esophageal tract, gastrointestinal tract (including the intestine, stomach and the colon), tracheobronchial tract, urinary tract, biliary tract, vascular system, etc.

In some instances it may be desirable to design stents to include sufficient flexibility while maintaining sufficient radial force to open the body lumen at the treatment site. However, in some stents, the compressible and flexible properties that assist in stent delivery may also result in a stent that has a tendency to migrate from its originally deployed position. For example, stents that are designed to be positioned in the esophageal or gastrointestinal tract may have a tendency to migrate due to peristalsis (i.e., the involuntary constriction and relaxation of the muscles of the esophagus, intestine, and colon which push the contents of the canal therethrough). Additionally, the generally moist and inherently lubricious environment of the esophagus, intestine, colon, etc. further contributes to a stent's tendency to migrate when deployed therein.
US 2020/214858 A1 relates to a stent comprising an elongated tubular member having a longitudinal axis, the elongated tubular member comprising at least one knitted filament forming a plurality of twisted knit stitches with intermediate rung portions extending circumferentially between radially adjacent twisted knit stitches. Each twisted knit stitch may be interconnected with a longitudinally adjacent twisted knit stitch forming a series of linked stitches. The elongated tubular member may be configured to move between a collapsed configuration and an expanded configuration, wherein in the collapsed configuration the series of linked stitches form longitudinal columns and in the expanded configuration the series of linked stitches extend helically around the elongated tubular member.
US 2019/307586 A1 relates to a stent comprising an elongated tubular member having a first end and a second end and an intermediate region disposed therebetween. The elongated tubular member configured to move between a collapsed configuration and an expanded configuration. The elongated tubular member may comprise at least one twisted filament, such as a knitted filament having a plurality of twisted knit stitches with intermediate rung portions extending between adjacent twisted knit stitches, or a plurality of helical filaments twisted with a plurality of longitudinal filaments.
US 2009/187240 A1 relates to an intraluminal prosthesis including an outer three-dimensional (3D) anti-migration structure that is attached to the outer wall of a fully covered or partially covered stent to prevent migration and still allow stent removal at a later period of time. A method of manufacturing the intraluminal prosthesis includes attaching the anti-migration structure by usage of a polymer such as polyurethane.

Therefore, in some instances it may be desirable to design a stent with anti-migration features to reduce the stent's tendency to migrate. Examples of medical devices including anti-migration features are disclosed herein.

### SUMMARY

This disclosure provides design, material, manufacturing method, and use alternatives for medical devices. As an example, a stent includes an elongated tubular member expandable from a radially collapsed configuration to a radially expanded configuration, the elongate tubular member including at least one filament, the elongated tubular member including a first segment in which the at least one filament is knitted into a first knitted pattern providing the first segment with a first performance characteristic and a second segment in which the at least one filament is knitted into a second knitted pattern providing the second segment with a second performance characteristic different from the first performance characteristic. Either the first knitted pattern or the second knitted pattern includes an anti-migration feature.

Additionally or alternatively, the anti-migration feature may include a plurality of anti-migration loops formed from the at least one filament, and when the plurality of anti-migration loops are included as part of the first knitted pattern, the first segment may be adapted such that the plurality of anti-migration loops retract into the first knitted pattern in response to the stent being placed in axial tension.

The anti-migration feature includes a plurality of anti-migration loops formed from the at least one filament, and when the plurality of anti-migration loops are included as part of the second knitted pattern, the second may be is adapted such that the second segment retains the plurality of anti-migration loops in response to the stent being placed in axial tension.

Additionally or alternatively, the first segment may include a first plurality of rows in which the filament is knitted into the first knitting pattern, the first plurality of rows extending circumferentially around a longitudinal axis of the elongated tubular member.

Additionally or alternatively, at least some rows of the first plurality of rows may include a plurality of twisted knit stitches with intermediate rung portions extending between adjacent twisted knit stitches.

Additionally or alternatively, the second segment may include a second plurality of rows in which the filament may be knitted into the second knitting pattern, the second plurality of rows extending circumferentially around the longitudinal axis of the elongated tubular member.

Additionally or alternatively, at least some rows of the second plurality of rows may include a plurality of stitches in which each stitch may include a loop that passes around a corresponding loop in a previous row without twisting.

Additionally or alternatively, the elongated tubular member may further include a third segment in which the at least one filament may be knitted into a knitted pattern that is the same as the first knitted pattern or the second knitted pattern.

Additionally or alternatively, the first segment may be disposed between the second knitted segment and the third knitted segment, and the filament may be knitted into the second knitted pattern within the third segment.

Additionally or alternatively, the second segment may be disposed between the first knitted segment and the third knitted segment, and the filament may be knitted into the first knitted pattern within the third segment.

Additionally or alternatively, the anti-migration feature may include a flared end.

As another example, a stent includes an elongated tubular member expandable from a radially collapsed configuration to a radially expanded configuration, the elongate tubular member including at least one filament, the elongated tubular member including a first segment in which the at least one filament is knitted into a first knitted pattern and a second segment in which the at least one filament is knitted into a second knitted pattern. A plurality of anti-migration loops are formed from the at least one filament within the first segment. The first knitted pattern is adapted to enable elongation of the first segment when the elongated tubular member is subjected to axial tension, thereby allowing the plurality of anti-migration loops to retract into the first knitted pattern. The second knitted pattern is adapted to restrict elongation of the second segment when the elongated tubular member is subjected to axial tension.

Additionally or alternatively, the first segment, including the plurality of anti-migration loops, may be flared.

Additionally or alternatively, the second segment may be flared.

Additionally or alternatively, the stent may further include one or more additional segments, in addition to the first segment and the second segment, wherein in each of the one or more additional segments, the at least one filament may be knitted into knitting patterns matching either the first knitting pattern or the second knitting pattern.

Additionally or alternatively, the stent may further include a third segment, wherein the second segment may be positioned between the first segment and the third segment, wherein the one or more filaments may be knitted into the first knitting pattern in the third segment, and wherein the third segment may include a plurality of anti-migration loops formed from the at least one filament

In another example, a stent includes an elongated tubular member expandable from a radially collapsed configuration to a radially expanded configuration, the elongate tubular member including at least one filament, the elongated tubular member including a first segment in which the at least one filament is knitted into a first knitted pattern and a second segment in which the at least one filament is knitted into a second knitted pattern. A plurality of anti-migration loops are formed within the second segment from the at least one filament. The first knitted pattern is adapted to enable elongation of the first segment when the elongated tubular member is subjected to axial tension and the second knitted pattern is adapted to restrict elongation of the second segment when the elongated tubular member is subjected to tension, thereby retaining the plurality of anti-migration loops.

Additionally or alternatively, the first segment may be flared.

Additionally or alternatively, the second segment may be flared.

Additionally or alternatively, the stent may further include a third segment in which the one or more filaments may be knitted into the second knitting pattern, the third segment may include a plurality of anti-migration loops formed from the at least one filament.

The above summary of some embodiments is not intended to describe each disclosed embodiment or every implementation of the present disclosure. The Figures, and Detailed Description, which follow, more particularly exemplify these embodiments.

### BRIEF DESCRIPTION OF THE DRAWINGS

The invention may be more completely understood in consideration of the following detailed description of various embodiments of the invention in connection with the accompanying drawings, in which:
Figure 1 is a schematic view of an illustrative stent;
Figure 2 is a side view of a first illustrative knitting pattern that may be used in forming the stent of Figure 1;
Figure 3 is a side view of a second illustrative knitting pattern that may be used in forming the stent of Figure 1;
Figure 4 is an enlarged side view of a portion of the illustrative stent of Figure 1, including both the first illustrative knitting pattern of Figure 2 and the second illustrative knitting pattern of Figure 3;
Figure 5 is a schematic side view of an illustrative stent shown in a deployed configuration;
Figure 6 is a schematic side view of the illustrative stent of Figure 5, shown in a constrained configuration;
Figure 7 is a schematic side view of an illustrative stent shown in a deployed configuration;
Figure 8 is a schematic side view of the illustrative stent of Figure 7, shown in a constrained configuration;
Figure 9 is a schematic side view of an illustrative stent shown in a deployed configuration;
Figure 10 is a schematic side view of an illustrative stent shown in a deployed configuration;
Figure 11 is a schematic side view of the illustrative stent of Figure 9 or the illustrative stent of Figure 10, shown in a constrained configuration;
Figure 12 is a schematic end view of an illustrative stent in a relaxed configuration;
Figure 13 is a schematic end view of the illustrative stent of Figure 12, shown in a constrained configuration;
Figure 14 is a schematic side view of an illustrative stent shown in a deployed configuration;
Figure 15 is a schematic side view of an illustrative stent shown in a deployed configuration;
Figure 16 is a schematic side view of the illustrative stent of Figure 14 or the illustrative stent of Figure 15, shown in a constrained configuration.

While the disclosure is amenable to various modifications and alternative forms, specifics thereof have been shown by way of example in the drawings and will be described in detail. It should be understood, however, that the intention is not to limit the disclosure to the particular embodiments described. The invention is disclosed in the appended claims.

### DESCRIPTION

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

In some instances, it may be desirable to provide an endoluminal implant, or stent, that can deliver luminal patency in a patient with an esophageal stricture or other medical condition. Such stents may be used in patients experiencing dysphagia, sometimes due to esophageal cancer. An esophageal stent may allow a patient to maintain nutrition via oral intake during cancer treatment or palliation periods. Some stents have a woven or knitted configuration to provide good radial strength with minimal foreshortening which may be desirable in esophageal and trachea-bronchial applications as well as some post-bariatric surgery applications. While the embodiments disclosed herein are discussed with reference to esophageal stents, it is contemplated that the stents described herein may be used and sized for use in other locations such as, but not limited to: bodily tissue, bodily organs, vascular lumens, non-vascular lumens and combinations thereof, such as, but not limited to, in the coronary or peripheral vasculature, trachea, bronchi, colon, small intestine, biliary tract, urinary tract, prostate, brain, stomach and the like.

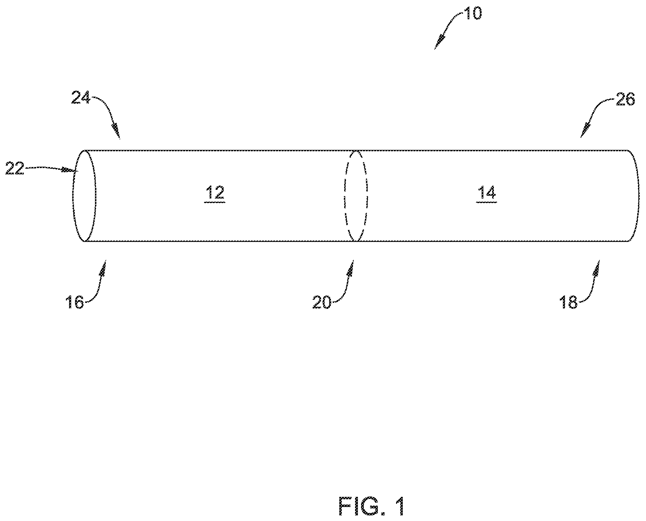

Figure 1 is a schematic view of an illustrative endoluminal implant 10, such as, but not limited to, a stent. In some instances, the stent 10 may take the form of an elongated tubular member including a first section 12 and a second section 14. While the stent 10 is described as generally tubular, it is contemplated that the stent 10 may take any cross-sectional shape desired. The stent 10 may have a first, or proximal end 16, a second, or distal end 18 and an intermediate region 20 that is disposed between the first end 16 and the second end 18. The stent 10 may include a lumen 22 that extends from a first opening adjacent the first end 16 to a second opening adjacent to the second end 18 to allow for the passage of food, fluids, etc. to pass therethrough.

The stent 10 may be expandable from a first radially collapsed configuration (not explicitly shown) to a second radially expanded configuration. In some cases, the stent 10 may be deployed to a configuration that is between the collapsed configuration and the expanded configuration, i.e., the stent 10 may be deployed having a deployed diameter that is greater than a diameter of the stent 10 or a particular portion thereof while in its collapsed configuration yet less than a diameter of the stent 10 or a particular portion thereof while in its fully expanded configuration. In some cases, the anatomy in which the stent 10 is deployed may influence its deployed configuration. For example, if the anatomy in which the stent 10 is to be deployed has a diameter that is less than a diameter of the stent 10 or a particular portion thereof when fully expanded, the stent 10 may have a deployed diameter that is intermediate its collapsed configuration diameter and its fully expanded configuration diameter.

The stent 10 may be formed of one or more, or a plurality of interwoven filaments. For example, the stent 10 may have a knitted structure, fabricated from one or more filaments interwoven with each other. In some cases, the stent 10 may have a knitted structure that is formed by knitting a single filament extending the entire length of the stent. In some cases, the stent 10 may be knitted from one or more filaments that are knitted together using two or more different knitting patterns. For example, the first section 12 may be formed by knitting together one or more filaments in a first knitting pattern while the second section 14 may be formed by knitting together one or more filaments in a second knitting pattern that is different from the first knitting pattern. The same filament(s) may be used throughout both the first knitting pattern in the first section 12 and the second knitting pattern in the second section 14, or different filaments may be used throughout each of the first knitting pattern in the first section 12 and the second knitting pattern in the second section 14. As will be discussed, each of the first knitting pattern and the second knitting pattern, and optionally any other knitting patterns used in forming the stent 10, may provide unique properties to the particular part of the stent 10 that is formed using those particular knitting patterns.

In some cases, the filament(s) of the stent 10 may be formed of a monofilament, while in other cases the stent 10 may be formed of two, three or more filaments that are wound, braided, or woven together prior to knitting the stent 10. In some instances, an inner surface and/or an outer surface of the stent 10 may be entirely, substantially, or partially covered with a polymeric covering or coating. The covering or coating may span the open spaces that are formed by the filament being knitted together. The covering or coating may help reduce food impaction and/or tumor or tissue ingrowth, for example.

It will be appreciated that in many cases, the performance demands on a stent, such as the stent 10, can be conflicting. For example, strength versus flexibility is a common conflict in designing medical devices such as stents. Constructing a stent that will remain in place, and not migrate, can conflict with a desire to possibly be able to move or even remove a stent. By forming the stent 10 having multiple sections such as the first section 12 and the second section 14 with differing knitting patterns, and thus with possibly differing performance characteristics, can help to provide the best of both worlds, as it were. As will be discussed subsequently, the knitting patterns disclosed herein can be used to form various sections or portions of the stent 10 in order to provide a desired combination of characteristics to the stent 10.

It is contemplated that the filament(s) of the stent 10 may be made from a number of different materials such as, but not limited to, metals, metal alloys, shape memory alloys and/or polymers, as desired, enabling the stent 10 to be expanded into shape when accurately positioned within the anatomy. In some instances, the material may be selected to enable the stent 10 to be removed with relative ease as well. For example, the stent 10 may be formed from alloys such as, but not limited to, Nitinol and Elgiloy^{®}. Depending on the material selected for construction of the stent 10, the stent 10 may be self-expanding, i.e., configured to automatically radially expand when unconstrained. In some instances, the stent 10 may not be self-expanding, and thus may not regain its fully expanded configuration without the assistance of an expansion device such as but not limited to an inflatable balloon disposed within the lumen 22. As used herein, the term "self-expanding" refers to the tendency of the stent 10 to return to a preprogrammed diameter when unrestrained by an external biasing force, e.g., a delivery catheter or sheath. While not shown, the stent 10 may include a one-way valve, such as an elastomeric slit valve or duck bill valve, positioned within the lumen 20 in order to prevent retrograde flow of gastrointestinal fluids, for example.

In some instances, in the radially expanded configuration, the stent 10 may include a first end region 24 proximate the first end 16 and a second end region 26 proximate the second end 18. In some cases, the first end region 24 and the second end region 28 may include retention features or anti-migration flared regions (not explicitly shown) having enlarged diameters relative to the intermediate region 20. The anti-migration flared regions, which may be positioned adjacent to the first end 16 and/or the second end 18, may be configured to engage an interior portion of the walls of the esophagus or other body lumen. In some cases, the retention features, or flared regions, may have a larger diameter than the intermediate region 20 of the stent 10 in order to prevent the stent 10 from migrating once placed in the esophagus or other body lumen. In some instances, a transition from the cross-sectional area of the intermediate region 20 to the retention features or flared regions may be gradual, sloped, or occur in an abrupt step-wise manner, as desired.

In some instances, the first anti-migration flared region may have a first outer diameter and the second anti-migration flared region may have a second outer diameter. In some instances, the first and second outer diameters may be approximately the same, while in other instances, the first and second outer diameters may be different. In some cases, the stent 10 may include only one or none of the anti-migration flared regions. For example, the first end region 24 may include an anti-migration flare while the second end region 26 may have an outer diameter similar to the intermediate region 20. It is further contemplated that the second end region 26 may include an anti-migration flare while the first end region 24 may have an outer diameter similar to an outer diameter of the intermediate region 20. In some embodiments, the stent 10 may have a uniform outer diameter from the first end 16 to the second end 18. In some embodiments, the outer diameter of the intermediate region 20 may be in the range of 15 to 25 millimeters in the fully expanded configuration. The outer diameter of the anti-migration flares may be in the range of 20 to 30 millimeters in the fully expanded configuration. It is contemplated that the outer diameter of the stent 10 may be varied to suit the desired application.

In some cases, composite filaments may be used to make the stent 10, which may include, for example, an outer shell or cladding made of Nitinol and a core formed of platinum or other radiopaque material. It is further contemplated that the stent 10 may be formed from polymers including, but not limited to, polyethylene terephthalate (PET). In some instances, the filaments of the stent 10, or portions thereof, may be bioabsorbable or biodegradable, while in other instances the filaments of the stent 10, or portions thereof, may be biostable.

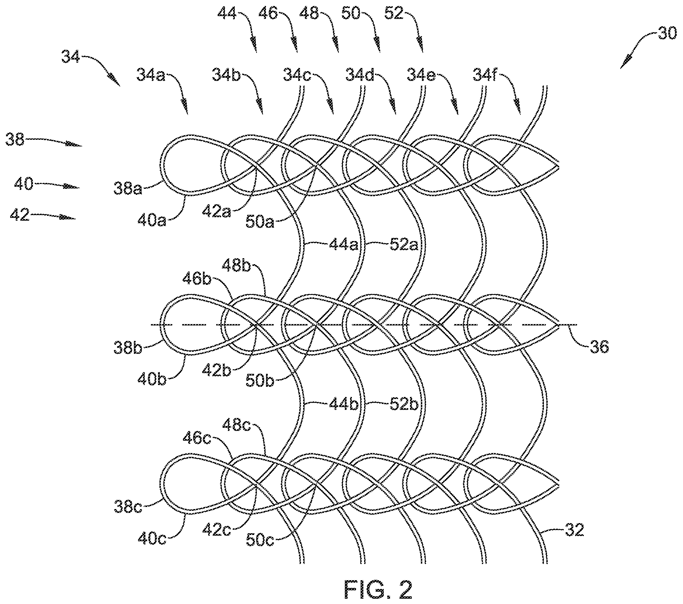

Figure 2 is a side view of a first illustrative knitting pattern 30 that may be used in forming part of the stent 10. In some cases, for example, the first knitting pattern 30 may be used in knitting the first section 12. The first knitting pattern 30 is formed by knitting a filament 32, row by row, with each row extending circumferentially around the stent 10. As shown, the first knitting pattern includes a plurality of rows 34, individually labeled as 34a, 34b, 34c, 34d, 34e and 34f, that extend circumferentially about the stent 10. The stent 10 may be considered as having a longitudinal axis 36. The stent 10 may include any number of rows 34 desired. For example, the number of rows 34 may be selected to achieve a desired length of the stent 10, or at least the desired length of the first section 12. The first knitting pattern 30 may be considered as including a plurality of twisted knit stitches.

The uppermost, or first, row 34a may be unsecured and active. In some instances, the first row 34a may include a plurality of loops 38, individually labeled as 38a, 38b, 38c. The loops 38 may each include a loop portion 40, individually labeled as 40a, 40b, 40c. The loops 38 may each include an overlapping base portion 42, individually labeled as 42a, 42b, 42c. The overlapping base portion 42 is understood to be the portion of the loops 38 in which one segment of the filament 32 overlaps or crosses over a second segment of the filament 32, with the segment of the filament forming the loop portion 40 extending therebetween. Adjacent loops 38 may be interconnected by a rung section 44, individually labeled as 44a, 44b. For example, a first rung section 44a may extend between the base portion 42a of the first loop 38a and the second base portion 42b of the second loop 38b.

The next row 34b may be suspended from the loops 38 of the first row 34a. For example, the second row 34b may include a plurality of loops 46, individually labeled as 46a, 46b, 46c. Each loop 46 includes a loop portion 48, individually labeled as 48a, 48b, 48c, and a base portion 50, individually labeled as 50a, 50b, 50c. Adjacent loops 46 may be interconnected by a rung section 52, individually labeled as 52a, 52b. As the stent 10, or at least the first section 12, is knitted, the loop portion 48 may be wrapped around the base portion 42 of the preceding row 34a. Thus, each loop 46 of a given row may be wrapped around the base portion 42 of a loop 38 of the preceding row.

It is contemplated that a single row 34 may be formed at a time. For example, the rows 34 may be formed in succession with a subsequent row, e.g., row 34b, being formed after the preceding row, e.g., row 34a, has formed a complete rotation about the stent 10. While not explicitly shown, the loops 38 of the first row 34a may be wrapped about a section of the filament 32 free from loops. As described herein, the loops 46 of the second row 34b may be wrapped about the base portion 42 of the loops 38 of the preceding row 34a. For example, the filament 32 may be knitted such that it extends from the first rung section 52a, is wrapped about the base portion 42a of the preceding row 34a, crosses back over itself to form base section 50b and continues to the next rung section 52b.

The first knitting pattern 30 provides the stent 10, or at least the first section 12 formed using the first knitting pattern 30, with particular performance characteristics. In some cases, the first knitting pattern 30 provides the stent 10, or at least the first section 12, with good radial strength and axial flexibility. The first knitting pattern 30 provides the stent 10, or at least the first section 12, with an ability to easily lengthen in response to the stent 10 being placed into tension. The loops such as the loops 38 and the loops 46 lengthen as the corresponding rungs 44 and 52 shorten, respectively. In cases where the first knitting pattern 30 includes one or more anti-migration loops, which are easily formed when knitting the first knitting pattern 30, applying a tensile force to the stent 10 causes the first section 12 to elongate, and causes the anti-migration loops to pull out. This means that the stent 10 can include anti-migration loops, in addition to or instead of including one or more flared retention regions, yet allow easy repositioning or even removal of the stent 10 by simply applying a tensile force to the first section 12, thereby causing the stent 10 to elongate and to pull out the anti-migration loops. Additional details regarding the first knitting pattern 30 may be found in US 2019/0307586.

In some applications, the stent 10 may be positioned within the anatomy at a location in which the stent 10 may be subjected to significant peristaltic motion. In situations such as this, the flexibility enabled by the first knitting pattern 30 may permit the stent 10 to bend and flex in response to the underlying pressure waves without causing the stent 10 to move within the anatomy. In some applications, there may be a desire to provide the stent 10 with other properties, such as but not limited to anti-migration loops that do not easily pull out in response to a tensile force.

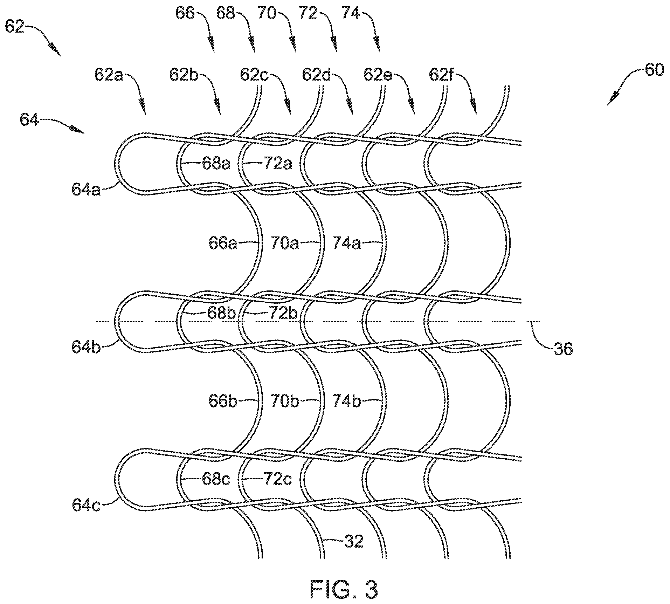

Figure 3 is a side view of a second illustrative knitting pattern 60 that may be used in forming part of the stent 10. In some cases, for example, the second knitting pattern 60 may be used in knitting the second section 14. The second knitting pattern 60 is formed by knitting a filament 32, row by row, with each row extending circumferentially around the stent 10. As shown, the second knitting pattern includes a plurality of rows 62, individually labeled as 62a, 62b, 62c, 62d, 62e and 62f, that extend circumferentially about the stent 10. The stent 10 may be considered as having a longitudinal axis 36. The stent 10 may include any number of rows 62 desired. For example, the number of rows 62 may be selected to achieve a desired length of the stent 10, or at least the desired length of the second section 14.

The uppermost, or first, row 62a may be unsecured and active. In some instances, the first row 62a may include a plurality of open loops 64, individually labeled as 64a, 64b, 64c. Each open loop 64 is joined to an adjoining open loop 64 via a rung 66, individually labeled as 66a and 66b. The next row, row 62b, includes a plurality of open loops 68, individually labeled as 68a, 68b, 68c. Each open loop 68 is joined to an adjoining loop open 68 via a rung 70, individually labeled as 70a and 70b. The next row, row 62c, includes a plurality of open loops 72, individually labeled as 72a, 72b, 72c. Each open loop 72 is joined to an adjoining loop 72 via a rung 74, individually labeled as 74a and 74b. An open loop, is a loop in which the base of the loop is not twisted (i.e., does not include segments if the filament forming the loop that cross over one another).

In forming the second knitting pattern 60, the filament 32 (a length of the filament 32 forming the first knitting pattern 30) forms a first row of a plurality of open loops 64 with rungs 66 extending between adjacent open loops 64. In the next successive circumferential row, the open loops 68 extend around the open base of the open loops 64 of the previous row, with rungs 70 extending between adjacent open loops 68. In the next successive circumferential row, the open loops 72 extend around the open base of the open loops 68 of the previous row. This pattern continues, with each successive circumferential row until sufficient rows 62 are completed so that the stent 10, or at least the second section 14, has achieved a desired length.

The second knitting pattern 60 provides the stent 10, or at least the second section 14 formed using the second knitting pattern 60, with particular performance characteristics. For example, the second knitting pattern 60 does not readily purse down or radially constrain when being removed or repositioned. This is because connected loops need to share a single rung in order to elongate. Each loop is on contention, which thus negates the ability of the second knitting pattern 60 to elongate, or at least to substantially elongate. As a result, if the second section 14 includes anti-migration loops, the anti-migration loops do not pull out in response to applying a tensile force to the stent 10.

Because the first knitting pattern 30 and the second knitting pattern 60 have different performance characteristics, including how they respond to an applied tensile strength and what that means for any anti-migration loops formed within either of the first knitting pattern 30 or the second knitting pattern 60, there can be advantages to using both the first knitting pattern 30 and the second knitting pattern 60 within the same stent 10.

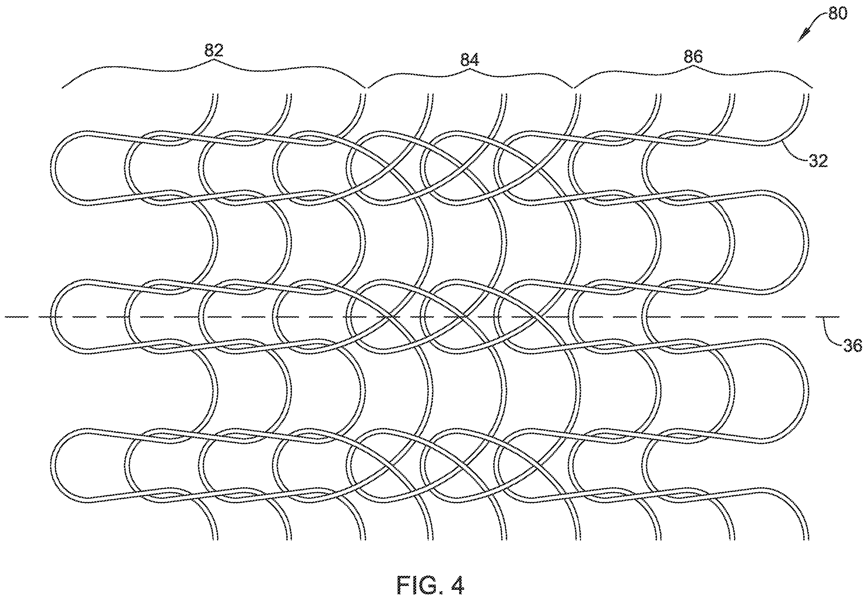

Figure 4 shows an illustrative composite knitting pattern 80 that includes a first segment 82, a second segment 84 and a third segment 86, together formed from a single filament 32. As used herein, the term "segment" is intended to refer to a tubular section of the stent having a longitudinal length. The stent is formed of a plurality of segments (i.e., tubular sections) extending end-to-end along the length of the stent. Thus, in the illustrated example, the stent includes the second segment 84 positioned longitudinally between the first segment 82 and the third segment 86. As illustrated, the second segment 84 employs the first knitting pattern 30 while the first segment 82 and the third segment 86 both employ the second knitting pattern 60. Because the first knitting pattern 30 and the second knitting pattern 60 are both knitted from the same single filament 32, it is possible to mix and match the first knitting pattern 30 and the second knitting pattern 60 within the stent 10 as desired (such as in adjacent tubular segments), in order to provide the stent 10 with appropriate characteristics.

In some cases, a stent formed using the composite knitting pattern 80 may include only a relatively small second segment 84 employing the first knitting pattern 30 while the first segment 82 and the third segment 86, both employing the second knitting pattern 60, may extend to the opposing ends of the stent. This would provide a stent that had two large segments that were less flexible, and less responsive to applied tensile forces, and one small segment that was more flexible and more responsive to applied tensile forces. It will be appreciated that the composite knitting pattern 80 shown in Figure 4 is merely illustrative, as any number of combinations of the first knitting pattern 30 and the second knitting pattern 60 may be combined within a single stent, depending on desired characteristics. It will also be appreciated that additional knitting patterns not expressly described herein may be combined in any number of combinations with the first knitting pattern 30 and the second knitting pattern 60, for example.

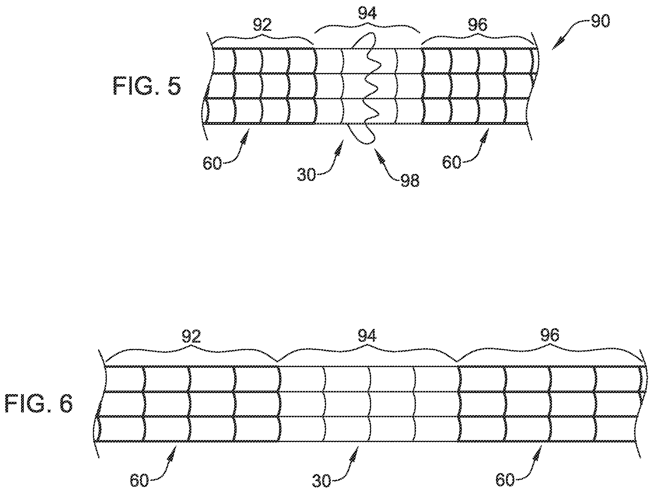

Figures 5 through 16 provide illustrative but non-limiting examples of combining the first knitting pattern 30 and the second knitting pattern 60 in various ways, along with various options for which the first knitting pattern 30 and/or the second knitting pattern 60 includes anti-migration loops. In each embodiment, it is understood each segment formed of the first knitting pattern 30 and each segment formed of the second knitting pattern 60 may be formed of a single filament 32 knitted into the respective knitting pattern. Thus, the filament 32 forming the first knitting pattern 30 and the second knitting pattern 60 may extend throughout the entire length of the stent. In Figures 5 through 16, the first knitting pattern 30 is represented in a standard weight line while the second knitting pattern 60 is represented in a heavier weight line.

Figure 5 is a schematic illustration of a segment of an illustrative stent 90 that includes a first segment 92 employing the second knitting pattern 60, a second segment 94 employing the first knitting pattern 30 and a third segment 96 employing the second knitting pattern 60. Thus, in the illustrated example, the stent 90 includes the second segment 94 positioned longitudinally between the first segment 92 and the third segment 96. In some ways, the stent 90 may be considered as an example of using the composite knitting pattern 80 shown in Figure 4. The second segment 94 includes a plurality of anti-migration loops 98 formed from the filament 32. It will be appreciated that the anti-migration loops 98 may be easily formed while knitting the stent 90 and/or subsequently formed. For example, a mandrel, such as an adjustable mandrel with adjustable features, can be used to displace the filament 32 radially outward from adjacent loops of the first knitting pattern 30 to form the filament into anti-migration loops. The anti-migration loops may be sections of the filament 32 that extend radially outward away from adjacent portions of the first knitting pattern 30. For example, the anti-migration loops 98 may be formed by extending a selected rung portion of the filament 32 that extends between adjacent loop portions radially outward beyond adjacent portions of the first knitting pattern 30. Further details regarding forming anti-migration loops may be found in US 2019/0029850.

Figure 6 represents the stent 90 after an axial tensile force has been applied to the stent 90. As can be seen, the first segment 92 and the third segment 96, both employing the second knitting pattern 60 have longitudinally lengthened somewhat. The anti-migration loops 98, which were present in the second segment 94, however, , can be seen to have pulled out and essentially disappeared. In other words, an axial force on the stent 90 causes the anti-migration loops 98 to be pulled into the knitting pattern 30, thus retracting the anti-migration loops 98. Accordingly, the stent 90 enjoys the characteristics both of the second knitting pattern 60, but also the first knitting pattern 30. The anti-migration loops 98 help to secure the stent 90 in place, but grasping the stent 90 in order to retrieve the stent 90 causes the stent 90 to longitudinally lengthen somewhat, with the second segment 94 allowing the anti-migration loops 98 to be retracted into the first knitting pattern 30. This means the stent 90 can easily be removed without damage to surrounding tissue that could otherwise occur if the anti-migration loops 98 were still engaged in tissue.

Use of a stent such as the stent 90 may be beneficial for treatment of conditions in which the stent may benefit from the presence of anti-migration loops such as the anti-migration loops 98, but device removal would be sensitive to the continued presence of the anti-migration loops 98. An example would be bariatric surgical leak treatments. By placing the anti-migration loops 98 within the second segment 94, which represents the first knitting pattern 30, this problem is avoided.

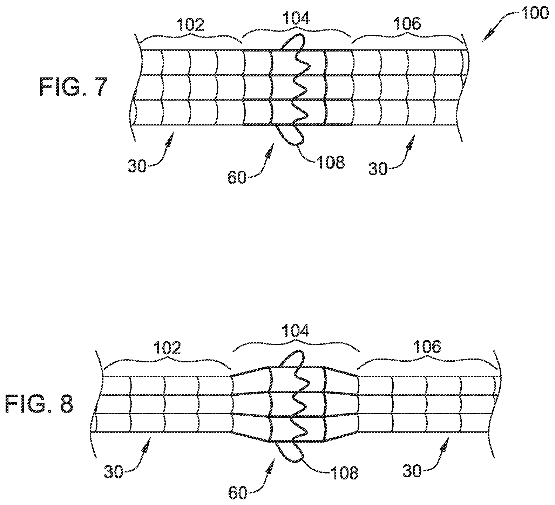

Figure 7 is a schematic illustration of a portion of an illustrative stent 100 that includes a first segment 102 employing the first knitting pattern 30, a second segment 104 employing the second knitting pattern 60 and a third segment 106 employing the first knitting pattern 30. Thus, in the illustrated example, the stent 100 includes the second segment 104 positioned longitudinally between the first segment 102 and the third segment 106. In some ways, the stent 100 may be considered as an example of an inverse of the stent 90. The second segment 104 includes a plurality of anti-migration loops 108 formed from the filament 32. It will be appreciated that the anti-migration loops 108 may be easily formed while knitting the stent 100 and/or subsequently formed. For example, a mandrel, such as an adjustable mandrel with adjustable features, can be used to displace the filament 32 radially outward from adjacent loops of the second knitting pattern 60 to form the filament into anti-migration loops. The anti-migration loops may be sections of the filament 32 that extend radially outward away from adjacent portions of the second knitting pattern 60. For example, the anti-migration loops 108 may be formed by extending a selected rung portion of the filament 32 that extends between adjacent loop portions radially outward beyond adjacent portions of the second knitting pattern 60.

Figure 8 represents the stent 100 after an axial tensile force has been applied to the stent 100. As can be seen, the first segment 102 and the third segment 106, both employing the first knitting pattern 30 have longitudinally lengthened. The second segment 104, however, which included the anti-migration loops 108, is largely unchanged from what is seen in Figure 7. Use of a stent such as the stent 100 may be beneficial in locations where the stent will be subject to substantial movement within the anatomy, such as peristaltic motion. The stent 100 is able to withstand pressure waves by allowing the segments of the stent including the first knitting pattern 30 to axially elongate while maintaining the radially extending anti-migration loops 108 even when the stent 100 is constrained. The stent 100 may be useful in highly motile regions such as esophageal strictures, for example. The ability of the stent 100 to remain intact while subjected to continual peristaltic motion and even to slightly deform through partial elongation may help prevent migration, risk of perforation and potential stent end stenosis. Rigid anti-migration loops 108, formed within the second knitting pattern 60 negates a migratory tendency.

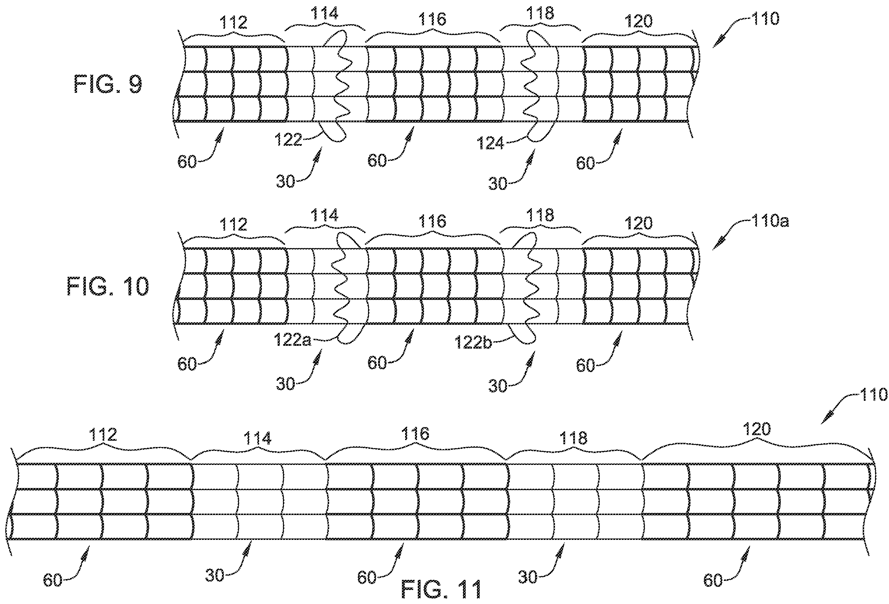

In some cases, a stent may include multiple regions formed of the first knitting pattern 30, with each of the multiple regions including anti-migration loops. Figure 9 is a schematic illustration of a portion of a stent 110 that includes a first segment 112 employing the second knitting pattern 60, a second segment 114 employing the first knitting pattern 30, a third segment 116 employing the second knitting pattern 60, a fourth segment 118 employing the first knitting pattern 30, and a fifth segment 120 employing the second knitting pattern 60. Thus, in the illustrated example, the stent 110 includes the second segment 114 positioned longitudinally between the first segment 112 and the third segment 116, the fourth segment 118 positioned longitudinally between the third segment 116 and the fifth segment 120, and the third segment 116 positioned longitudinally between the second segment 114 and the fourth segment 118. The second segment 114 includes a plurality of anti-migration loops 122 that extend radially outward and are pointed towards a center of the stent 110. The fourth segment 118 includes a plurality of anti-migration loops 124 that also extend radially outward and point toward the center of the stent 110, with the anti-migration loops 122 in the second segment 114 pointing in the opposite direction from the anti-migration loops 124 in the fourth segment 118.

Figure 10 is a schematic illustration of a segment of an illustrative stent 110a that includes a first segment 112 employing the second knitting pattern 60, a second segment 114 employing the first knitting pattern 30, a third segment 116 employing the second knitting pattern 60, a fourth segment 118 employing the first knitting pattern 30 and a fifth segment 120 employing the second knitting pattern 60. The second segment 114 includes a plurality of anti-migration loops 122a that extend radially outward and are pointed away from a center of the stent 110. The fourth segment 118 includes a plurality of anti-migration loops 124a that also extend radially outward and point away from the center of the stent 110, with the anti-migration loops 122a in the second segment 114 pointing in the opposite direction from the anti-migration loops 124a in the fourth segment 118.

Because the stent 110 and the stent 110a have their anti-migration loops 122, 122a and 124, 124a formed within portions of the stent 110, 110a that employ the first knitting pattern 30, it will be appreciated that Figure 11 may be considered as representing either the stent 110 or the stent 110a in its constrained configuration, with an axial tensile force applied to the stent 110, 110a.

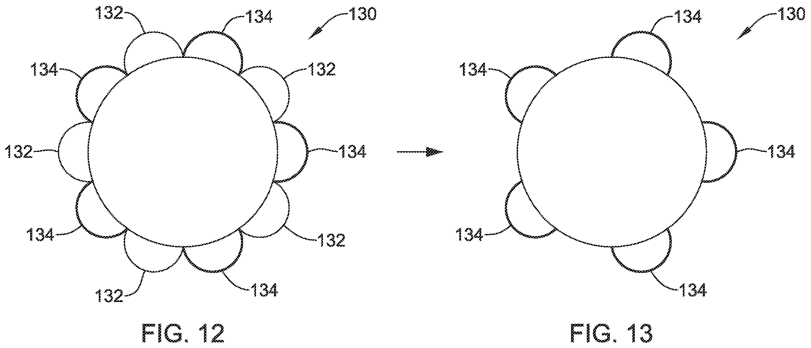

Figure 12 is a schematic end view of an illustrative stent 130 in which every second loop on the cross-section is formed from an alternate knitting pattern. For example, the stent 130 may include a number of anti-migration loops 132 that are formed as part of the first knitting pattern 30 and a number of anti-migration loops 134 that are formed as part of the second knitting pattern 60. The anti-migration loops 132 and the anti-migration loops 134 may be arranged in any desired arrangement and/or placement along the length and/or around the circumference of the stent 130. For example, the anti-migration loops 132 may alternate with the anti-migration loops 134 around the circumference of the stent 130, for example. In some instances, the anti-migration loops 132 may alternate with the anti-migration loops 134 in a single row, in successive rows, or in adjacent segments of the stent 130, for example. Each row may have the same pattern of alternating anti-migration loops 132 and anti-migration loops 134, for example. In some cases, the pattern may vary from row to row, as desired. For example, in some instances a single row of the stent may include a portion having the first knitting pattern 30 and a portion having the second knitting pattern 60. Such a row may include one or more anti-migration loops 132 formed in the first knitting pattern 30 and/or one or more anti-migration loops 134 formed in the second knitting pattern 60. In some cases, the anti-migration loops 132 and/or the anti-migration loops 134 in a particular row may be circumferentially aligned or circumferentially offset from the anti-migration loops 132 and/or the anti-migration loops 134 in another row, for example.

As seen in Figure 13, when the stent 130 is axially elongated, the anti-migration loops 132 that are part of the first knitting pattern 30 will retract into the first knitting pattern 30 during axial elongation of the stent 130 while the anti-migration loops 134 that are part of the second knitting pattern 60 will not retract into the second knitting pattern 60 during axial elongation. The resulting configuration, with half as many anti-migration loops, may be easier to move or remove from the anatomy.

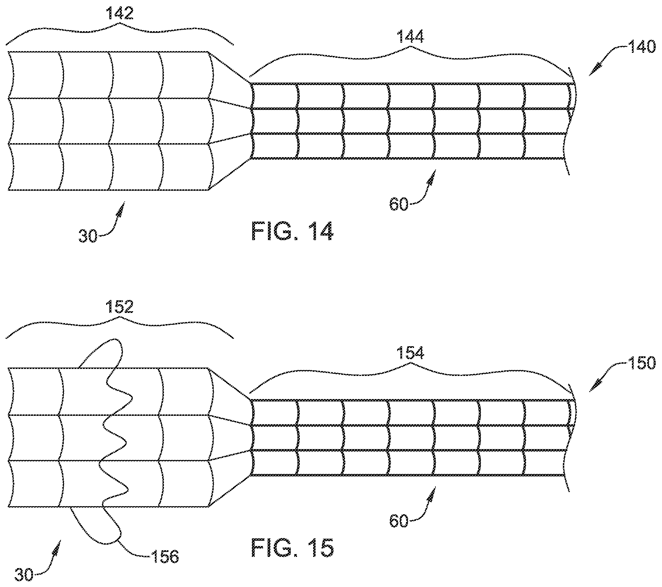

Figure 14 is a schematic side view of an illustrative stent 140. The illustrative stent 140 includes a first segment 142 that employs the first knitting pattern 30 and a second segment 144 that employs the second knitting pattern 60. In the illustrated example, the second segment 144 is positioned longitudinally adjacent to the first segment 142. The first segment 142 is flared, and thus has a larger diameter than does the second segment 144. It will be appreciated that applying an axial tensile force to the stent 140 will cause the flared first segment 142 to reduce in diameter and longitudinally elongate, essentially removing the flare in order to make repositioning or even removal of the stent 140 easier.

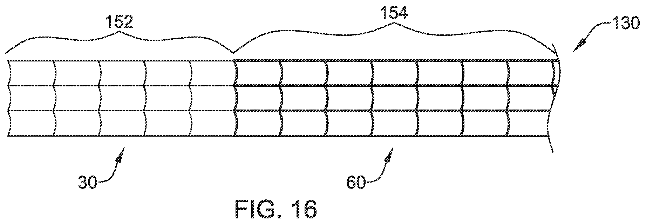

Figure 15 is a schematic side view of an illustrative stent 150 that includes a first segment 152 employing the first knitting pattern 30 and a second segment 154 employing the second knitting pattern 60. In the illustrated example, the second segment 154 is positioned longitudinally adjacent to the first segment 152. The first segment 152 is flared, and thus has a larger diameter than does the second segment 154. The first segment 152 also includes a plurality of anti-migration loops 156. It will be appreciated that applying an axial tensile force to the stent 150 will cause the flared first segment 152 to reduce in diameter and longitudinally elongate, essentially removing the flare and causing the anti-migration loops 156 to retract into the first knitting pattern 30 in the first segment 152, thereby making repositioning or even removal of the stent 150 easier. This can be seen in Figure 16.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. A stent (10), comprising:
an elongated tubular member expandable from a radially collapsed configuration to a radially expanded configuration, the elongate tubular member comprising at least one filament (32), the elongated tubular member including a first segment (82) in which the at least one filament (32) is knitted into a first knitted pattern (30) providing the first segment (82) with a first performance characteristic and a second segment (84) in which the at least one filament (32) is knitted into a second knitted pattern (60) providing the second segment (84) with a second performance characteristic different from the first performance characteristic;
wherein either the first knitted pattern (30) or the second knitted pattern (60) includes an anti-migration feature;
**characterised in that**
the anti-migration feature comprises a plurality of anti-migration loops (98) formed from the at least one filament (32), and when the plurality of anti-migration loops (98) are included as part of the second knitted pattern (60), the second segment (84) is adapted such that the second segment (84) retains the plurality of anti-migration loops (98) in response to the stent (10) being placed in axial tension.

2. The stent (10) of claim 1, wherein, when the plurality of anti-migration loops (98) are included as part of the first knitted pattern (30), the first segment (82) is adapted such that the plurality of anti-migration loops (98) retract into the first knitted pattern (30) in response to the stent (10) being placed in axial tension.

3. The stent (10) of any one of claims 1 or 2, wherein the first segment (82) comprises a first plurality of rows in which the filament (32) is knitted into the first knitting pattern (30), the first plurality of rows extending circumferentially around a longitudinal axis of the elongated tubular member.

4. The stent (10) of claim 3, wherein at least some rows of the first plurality of rows comprise a plurality of twisted knit stitches with intermediate rung portions extending between adjacent twisted knit stitches.

5. The stent (10) of any one of claims 1 to 4, wherein the second segment (84) comprises a second plurality of rows in which the filament (32) is knitted into the second knitting pattern (60), the second plurality of rows extending circumferentially around the longitudinal axis of the elongated tubular member.

6. The stent (10) of any one of claims 1 to 5, wherein the elongated tubular member further comprises a third segment (86) in which the at least one filament (32) is knitted into a knitted pattern that is the same as the first knitted pattern (30) or the second knitted pattern (60).

7. The stent (10) of claim 6, wherein the first segment (82) is disposed between the second knitted segment and the third knitted segment, and the filament (32) is knitted into the second knitted pattern (60) within the third segment (86).

8. The stent (10) of claim 6, wherein the second segment (84) is disposed between the first knitted segment and the third knitted segment, and the filament (32) is knitted into the first knitted pattern (30) within the third segment (86).

9. The stent (10) of any one of claims 1 to 8, wherein the anti-migration feature comprises a flared end.

## Patentansprüche

1. Stent (10), der aufweist:
ein längliches Röhrenteil, das aus einer radial kollabierten Konfiguration in eine radial expandierte Konfiguration expandierbar ist, wobei das längliche Röhrenteil mindestens ein Filament (32) aufweist und das längliche Röhrenteil aufweist: ein erstes Segment (82), in dem das mindestens eine Filament (32) zu einem ersten Strickmuster (30) gestrickt ist, das das erste Segment (82) mit einem ersten Leistungskennwert versieht, und ein zweites Segment (84), in dem das mindestens eine Filament (32) zu einem zweiten Strickmuster (60) gestrickt ist, das das zweite Segment (84) mit einem zweiten Leistungskennwert versieht, der sich vom ersten Leistungskennwert unterscheidet;
wobei das erste Strickmuster (30) oder das zweite Strickmuster (60) ein Anti-Migrations-Merkmal aufweist;
**dadurch gekennzeichnet, dass**
das Anti-Migrations-Merkmal mehrere Anti-Migrations-Schlaufen (98) aufweist, die aus dem mindestens einen Filament (32) gebildet sind, und, wenn die mehreren Anti-Migrations-Schlaufen (98) als Teil des zweiten Strickmusters (60) enthalten sind, das zweite Segment (84) so angepasst ist, dass das zweite Segment (84) die mehreren Anti-Migrations-Schlaufen (98) als Reaktion darauf beibehält, dass der Stent (10) in Axialspannung versetzt wird.

2. Stent (10) nach Anspruch 1, wobei, wenn die mehreren Anti-Migrations-Schlaufen (98) als Teil des ersten Strickmusters (30) enthalten sind, das erste Segment (82) so angepasst ist, dass sich die mehreren Anti-Migrations-Schlaufen (98) in das erste Strickmuster (30) als Reaktion darauf zurückziehen, dass der Stent (10) in Axialspannung versetzt wird.

3. Stent (10) nach Anspruch 1 oder 2, wobei das erste Segment (82) mehrere erste Reihen aufweist, in denen das Filament (32) zum ersten Strickmuster (30) gestrickt ist, wobei sich die mehreren ersten Reihen entlang des Umfangs um eine Längsachse des länglichen Röhrenteils erstrecken.

4. Stent (10) nach Anspruch 3, wobei mindestens einige Reihen der mehreren ersten Reihen mehrere gedrehte Strick-Maschen mit Querfadenzwischenabschnitten aufweisen, die sich zwischen benachbarten gedrehten Strick-Maschen erstrecken.

5. Stent (10) nach einem der Ansprüche 1 bis 4, wobei das zweite Segment (84) mehrere zweite Reihen aufweist, in denen das Filament (32) zum zweiten Strickmuster (60) gestrickt ist, wobei sich die mehreren zweiten Reihen entlang des Umfangs um die Längsachse des länglichen Röhrenteils erstrecken.

6. Stent (10) nach einem der Ansprüche 1 bis 5, wobei das längliche Röhrenteil ferner ein drittes Segment (86) aufweist, in dem das mindestens eine Filament (32) zu einem Strickmuster gestrickt ist, das das gleiche ist wie das erste Strickmuster (30) oder das zweite Strickmuster (60).

7. Stent (10) nach Anspruch 6, wobei das erste Segment (82) zwischen dem zweiten gestrickten Segment und dem dritten gestrickten Segment angeordnet ist und das Filament (32) zum zweiten Strickmuster (60) im dritten Segment (86) gestrickt ist.

8. Stent (10) nach Anspruch 6, wobei das zweite Segment (84) zwischen dem ersten gestrickten Segment und dem dritten gestrickten Segment angeordnet ist und das Filament (32) zum ersten Strickmuster (30) im dritten Segment (86) gestrickt ist.

9. Stent (10) nach einem der Ansprüche 1 bis 8, wobei das Anti-Migrations-Merkmal ein aufgeweitetes Ende aufweist.

## Revendications

1. Stent (10) comprenant:
un élément tubulaire allongé extensible d'une configuration radialement repliée à une configuration radialement déployée, l'élément tubulaire allongé comprenant au moins un filament (32), l'élément tubulaire allongé incluant un premier segment (82) dans lequel le au moins un filament (32) est tricoté selon un premier motif tricoté (30) conférant au premier segment (82) une première caractéristique de performance et un deuxième segment (84) dans lequel le au moins un filament (32) est tricoté selon un deuxième motif tricoté (60) conférant au deuxième segment (84) une deuxième caractéristique de performance différente de la première caractéristique de performance;
dans lequel le premier motif tricoté (30) ou le deuxième motif tricoté (60) inclut une caractéristique anti-migration;
**caractérisé en ce que** la caractéristique anti-migration comprend une pluralité de boucles anti-migration (98) formées à partir du au moins un filament (32), et lorsque la pluralité de boucles anti-migration (98) sont incluses en tant que partie du deuxième motif tricoté (60), le deuxième segment (84) est adapté de telle sorte que le deuxième segment (84) retient la pluralité de boucles anti-migration (98) en réponse au fait que le stent (10) est placé en tension axiale.

2. Stent (10) selon la revendication 1, dans lequel, lorsque la pluralité de boucles anti-migration (98) sont incluses en tant que partie du premier motif tricoté (30), le premier segment (82) est adapté de telle sorte que la pluralité de boucles anti-migration (98) se rétractent dans le premier motif tricoté (30) en réponse au fait que le stent (10) est placé en tension axiale.

3. Stent (10) selon l'une quelconque des revendications 1 ou 2, dans lequel le premier segment (82) comprend une première pluralité de rangées dans lesquelles le filament (32) est tricoté selon le premier motif de tricotage (30), la première pluralité de rangées s'étendant circonférentiellement autour d'un axe longitudinal de l'élément tubulaire allongé.

4. Stent (10) selon la revendication 3, dans lequel au moins certaines rangées de la première pluralité de rangées comprennent une pluralité de mailles tricotées torsadées avec des parties d'échelon intermédiaires s'étendant entre les mailles tricotées torsadées adjacentes.

5. Stent (10) selon l'une quelconque des revendications 1 à 4, dans lequel le deuxième segment (84) comprend une deuxième pluralité de rangées dans lesquelles le filament (32) est tricoté selon le deuxième motif de tricot (60), la deuxième pluralité de rangées s'étendant circonférentiellement autour de l'axe longitudinal de l'élément tubulaire allongé.

6. Stent (10) selon l'une quelconque des revendications 1 à 5, dans lequel l'élément tubulaire allongé comprend en outre un troisième segment (86) dans lequel le au moins un filament (32) est tricoté selon un motif tricoté qui est identique au premier motif tricoté (30) ou au deuxième motif tricoté (60).

7. Stent (10) selon la revendication 6, dans lequel le premier segment (82) est disposé entre le deuxième segment tricoté et le troisième segment tricoté, et le filament (32) est tricoté selon le deuxième motif tricoté (60) dans le troisième segment (86).

8. Stent (10) selon la revendication 6, dans lequel le deuxième segment (84) est disposé entre le premier segment tricoté et le troisième segment tricoté, et le filament (32) est tricoté selon le premier motif tricoté (30) dans le troisième segment (86).

9. Stent (10) selon l'une quelconque des revendications 1 à 8, dans lequel la caractéristique anti-migration comprend une extrémité évasée.
